# EUROPÄISCHE PATENTSCHRIFT

(11) **EP 4 252 918 B1**
(45) Veröffentlichungstag und Bekanntmachung des Hinweises auf die Patenterteilung: **16.07.2025**
(21) Anmeldenummer: 22164746.4
(22) Anmeldetag: 28.03.2022
(51) Int. Cl.: B05B 11/00, B05B 15/30, B05B 11/10

(54) **FLÜSSIGKEITSSPENDER MIT ZWEI FLÜSSIGKEITSSPEICHERN**
FLUID DISPENSER WITH TWO FLUID STORAGE UNITS
DISTRIBUTEUR DE LIQUIDE POURVU DE DEUX RÉSERVOIRS DE LIQUIDE

(43) Veröffentlichungstag der Anmeldung: 04.10.2023
(73) Patentinhaber: Aptar Radolfzell GmbH, 78315 Radolfzell (DE)
(72) Erfinder: Jung, Timo, 78315 Radolfzell (DE); Pfeffer, Hubertus, 78253 Eigeltingen (DE); Schmid, Lenz, 78467 Konstanz (DE)
(74) Vertreter: Witte, Weller und Partner Patentanwälte mbB Stuttgart

(56) Entgegenhaltungen:
- WO-A1-2021/093972
- CN-U- 214 650 011
- CN-U- 214 876 887
- DE-U1- 202004 010 515
- US-A- 5 623 974
- US-A1- 2001 025 859
- US-A1- 2016 023 228

## Beschreibung

### ANWENDUNGSGEBIET UND STAND DER TECHNIK

Die Erfindung betrifft primär einen Flüssigkeitsspender zum Austrag einer insbesondere pharmazeutischen oder kosmetischen Flüssigkeit, die durch Vermengung von zwei oder mehr Ausgangsflüssigkeiten erzeugt wird. Dabei ist bei einem gattungsbildenden Flüssigkeitsspender vorgesehen, dass dieser für die Ausgangsflüssigkeiten jeweils einen eigenen Flüssigkeitsspeicher aufweist. Eine getrennte Lagerung der Ausgangsflüssigkeiten bis zum Zeitpunkt des Austrags kann abhängig von der Ausgangsflüssigkeiten geboten sein, da die direkte Lagerung der bereits vermengten Flüssigkeit in einem gemeinsamen Flüssigkeitsspeicher deren Qualität verschlechtern würde.

Im Zuge des Austrags werden bei einem gattungsbildenden Flüssigkeitsspeicher die Ausgangsflüssigkeiten durch eine gemeinsame Pumpeinrichtung oder durch separate Pumpeinrichtungen zu einer Austragöffnung gefördert und auf dem Weg miteinander vermengt.

Die Herstellung eines Flüssigkeitsspenders mit mehr als einem Flüssigkeitsspeicher erschwert die Befüllung der Flüssigkeitsspeicher. Es stellt in der Praxis ein Problem dar, ein Speicherbauteil mit zwei Flüssigkeitsspeichern zu befüllen und anschließend die Pumpeinrichtungen und oder den Austragkopf hieran zu montieren.

### AUFGABE UND LÖSUNG

Aufgabe der Erfindung ist es, einen Flüssigkeitsspender der gattungsgemäßen Art vorzuschlagen, der eine vorteilhafte Art der Befüllung gestattet. Aufgabe ist es weiterhin, ein hierfür passendes Befüllungsverfahren vorzuschlagen.

Erfindungsgemäß vorgeschlagen wird ein Flüssigkeitsspender zur Abgabe einer kosmetischen oder pharmazeutischen Flüssigkeit, die durch Vermengung zweier Ausgangsflüssigkeiten erzeugt wird.

Dieser Flüssigkeitsspender weist in der bereits beschriebenen Weise üblicherweise mindestens zwei Flüssigkeitsspeicher zur Aufnahme der zwei Ausgangsflüssigkeiten auf. Die Flüssigkeitsspeicher definieren eine Haupterstreckungsrichtung des Flüssigkeitsspenders. Insbesondere vorzugsweise sind die Wandungen der Flüssigkeitsspeicher zumindest abschnittsweise zylindrisch bezogen auf die Haupterstreckungsrichtung ausgebildet.

Die Flüssigkeitsspeicher werden vorzugsweise zumindest primär durch einen oder jeweils einen Hauptspeicherkörper gebildet, der die Flüssigkeitsspeicher gegenüber einer Umgebung isoliert. Zwischen den Flüssigkeitsspeichern ist mindestens eine Trennwandung vorgesehen, so dass die Ausgangsflüssigkeiten in den Flüssigkeitsspeichern sich nicht miteinander vermengen. Bei einer Bauweise, bei der zwei getrennte Hauptspeicherkörper jeweils einen Flüssigkeitsspeicher zum überwiegenden Teil definieren, sind mindestens zwei Trennwandungen zwischen den Flüssigkeitsspeichern vorgesehen.

Ein erfindungsgemäßer Flüssigkeitsspender weist mindestens eine Austragöffnung auf, durch die die vermengte Flüssigkeit ausgetragen wird. Denkbar sind auch Gestaltungen, bei denen getrennte Austragöffnungen vorgesehen sind und die Vermengung der Flüssigkeiten erst stromabwärts der Austragöffnungen während der Applikation erfolgt.

Weiterhin weist ein erfindungsgemäßer Flüssigkeitsspender mindestens eine Pumpeinrichtung auf, mittels derer Flüssigkeit aus den Flüssigkeitsspeichern zur Austragöffnung gefördert wird. Vorzugsweise ist je Flüssigkeitsspeicher eine eigene Pumpeinrichtung vorgesehen, wobei jedoch insbesondere vorzugsweise diese mindestens zwei Pumpeinrichtungen mechanisch gekoppelt sind, so dass sie stets zumindest phasenweise gleichzeitig betätigt werden. Vorzugsweise sind die beiden Ausgangsseiten der vorzugsweise mindestens zwei Pumpeinrichtungen mit einem gemeinsamen Vermengungskanal verbunden, in dem die Ausgangsflüssigkeiten zusammengeführt werden.

Der Flüssigkeitsspender weist vorzugsweise einen Betätigungsdrücker auf, der getrennt von der Austragöffnung oder gemeinsam mit der Austragöffnung gegenüber den Flüssigkeitsspeichern verlagerbar ist. Die mindestens eine Pumpeinrichtung ist hieran derart angekoppelt, dass die Verlagerung des Betätigungsdrückers zu einer Betätigung der Pumpeinrichtung führt.

Zur Entnahme der Flüssigkeit aus den Flüssigkeitsspeichern ist jeweils an einem oberen Ende, welches auf der Seite der mindestens einen Pumpeinrichtung und der mindestens einen Austragöffnung vorgesehen ist, jeweils eine Ansaugöffnung vorgesehen, durch die hindurch Flüssigkeit zur Pumpeinrichtung gesogen werden kann. An diesen mindestens zwei Ansaugöffnungen ist jeweils ein Ansaugrohr vorgesehen, welches sich bis zum gegenüberliegenden unteren Ende der Flüssigkeitsspeicher erstreckt.

Die Verwendung des Flüssigkeitsspenders findet in einer Ausrichtung statt, in der das untere Ende der Flüssigkeitsspeicher nach unten weist und das obere Ende nach oben. Die Ausgangsflüssigkeiten werden mittels der Pumpeinrichtungen aus den Flüssigkeitsspeichern angesogen, wobei die frei von den Absaugöffnungen in die Flüssigkeitsspeicher hineinragenden und dadurch von Flüssigkeit umgebenen Ansaugrohre verursachen, dass die Flüssigkeit vom unteren Ende der Flüssigkeitsspeicher entnommen wird.

Bei einem erfindungsgemäßen Flüssigkeitsspeicher ist vorgesehen, dass die Befüllung der Flüssigkeitsspeicher in einer umgedrehten Stellung mit nach oben weisendem unteren Ende der Flüssigkeitsspeicher erfolgt. Zu diesem Zweck ist in einem Bodenbereich der Flüssigkeitsspeicher am unteren Ende jeweils eine Füllöffnung vorgesehen.

Es hat sich herausgestellt, dass diese Art der Befüllung erheblich Vorteile bietet. So kann ein Erfindungsgemäßer Spender bis auf ein Speicherbodenbauteil oder Stopfen vollständig montiert werden, bevor die Befüllung stattfindet.

Die Füllöffnung kann dadurch gebildet sein, dass der gemeinsame Hauptspeicherkörper während der Befüllung an seinem unteren Ende noch offen ist, so dass durch diese offenen unteren Enden der mindestens zwei Flüssigkeitsspeicher die Flüssigkeit eingebracht werden kann und anschließend die offenen Enden mittels eines gemeinsamen oder mittels separater Stopfen oder mittels eines Speicherbodenbauteils verschlossen werden.

Die Flüssigkeitsspeicher weisen im Bodenbereich einen Speicherboden auf, wobei die Speicherböden beider Flüssigkeitsspeicher vorzugsweise durch ein gemeinsames Speicherbodenbauteil gebildet sind, welches insbesondere vorzugsweise flüssigkeitsdicht mit dem genannten Hauptspeicherkörper verbunden ist. Insbesondere können das Speicherbodenbauteil oder die Speicherbodenbauteile durch Verkleben oder Verschweißen mit dem Hauptspeicherkörper dicht verbunden sein. Auch eine einfache Pressverbindung kann ausreichen, wobei hierfür vorzugsweise der Hauptspeicherkörper in jeweils einer runden Öffnung endet, in die die entsprechend außenseitig runden Speicherbodenbauteile eingedrückt sind.

Bei einer Gestaltung mit einem oder mehreren Speicherbodenbauteilen sind die Füllöffnungen vorzugsweise als Durchbrechungen in diesem vorzugsweise gemeinsamen Speicherbodenbauteil ausgebildet.

Der Speicherboden kann vorzugsweise dem Zweck dienen, das jeweilige Ansaugrohr in einer Position zu halten, die beim Befüllen der Flüssigkeitsspeicher mit den Ausgangsflüssigkeiten verhindert, dass eine entsprechende Füllnadel mit den Ansaugrohren kollidiert.

Hierfür wird es als insbesondere vorteilhaft angesehen, wenn die Speicherböden jeweils eine Haltegeometrie aufweisen, durch die ein unteres Ende des jeweiligen Ansaugrohrs von der jeweiligen Füllöffnung ferngehalten wird. Der Speicherboden erstreckt sich zu diesem Zweck so weit in Richtung des oberen Endes der Flüssigkeitsspeicher, dass er bis in den Bereich des jeweiligen Ansaugrohres ragt und dieses mechanisch von der Einfüllöffnung fernhält. Insbesondere kann die Füllöffnung, die den Speicherboden durchbricht, eine die jeweilige Füllöffnung umgebenden Haltewandung aufweisen. Das Ansaugrohr ist auf Höhe der Haltewandung quer zur Haupterstreckungsrichtung versetzt zur Füllöffnung angeordnet und dadurch außerhalb der Haltewandung angeordnet. Es kann aufgrund der Haltewandung nicht bis in den Bereich der Füllöffnung gelangen.

Die Speicherböden sind jeweils zumindest abschnittsweise mit schräggestellten Leitflächen versehen. Durch diese können die Ansaugrohre während der Montage des Flüssigkeitsspenders, insbesondere während der Anbringung des entsprechend gestalteten Speicherbodenbauteils, von der jeweiligen Füllöffnung weggedrückt werden.

Alternativ zu den Speicherböden mit schräggestellten Leitflächen oder zusätzlich ist vorgesehen, dass im Bodenbereich der Flüssigkeitsspeicher Sperrabschnitte vorgesehen sind, die innerhalb des jeweiligen Flüssigkeitsspeichers fluchtend mit den jeweiligen Füllöffnungen angeordnet sind und somit zwischen den Füllöffnungen und den Ansaugöffnungen angeordnet sind. Ein im Wesentlichen gradliniges oder leicht gebogenes Ansaugrohr kann somit nicht in die Füllöffnung hineinragen, sondern wird vom Sperrabschnitt hiervon ferngehalten. Eine Oberseite der Sperrabschnitte ist vorzugsweise mit einer schräggestellten Ebene oder mit einer sich nach oben verjüngenden Form versehen, um das Ansaugrohr während der Montage zur Seite zu drücken.

Zugunsten einer einfachen Herstellung der Kunststoffeinzelteile können die Sperrabschnitte durch vom Speicherbodenbauteil getrennte Einsätze gebildet sein, die am Speicherbodenbauteil befestigt sind, insbesondere in den Füllöffnungen mittels einer Klemm- oder Rastverbindung.

Eine weitere mögliche Gestaltung zur Lagefixierung des Ansaugrohres bzw. zu seiner Positionierung abseits der Füllöffnung sieht vor, dass mittels eines von der Ansaugöffnung beabstandeten Haltemittels insbesondere im Bereich einer Mantelwandung des Flüssigkeitsspeichers das Ansaugrohr gesichert ist. Das Haltemittel kann beispielsweise an der Innenseite der Mantelwandung vorgesehen sein.

Durch die Sperrabschnitte und die beschriebene Gestaltung des Speicherbodens sowie durch da mantelwandungsseitige Haltemittel kann das Ansaugrohr wirksam von der Füllöffnung ferngehalten werden. Insbesondere gestatten es dies, die Ansaugöffnung, an der das Ansaugrohr angebracht ist, und die Füllöffnung in bezogen auf die Haupterstreckungsrichtung fluchtender Anordnung vorzusehen. Dies ist insbesondere bei schlanken länglichen Flüssigkeitsspendern von Vorteil.

Wenn die Formgebung der Flüssigkeitsspeicher es zulässt, kann es aber auch von Vorteil sein, die Ansaugöffnungen gegenüber den Füllöffnungen bezogen auf die Haupterstreckungsrichtung versetzt anzuordnen. Im Falle eines geradlinigen Ansaugrohres, welches sich von der Ansaugöffnung geradlinig nach unten erstreckt, kann hierdurch bewirkt oder begünstigt werden, dass das Ansaugrohr nicht in den Bereich der Füllöffnung gelangt und den Befüllungsvorgang hierdurch erschwert.

Nach der Befüllung werden die Füllöffnungen verschlossen, vorzugweise mittels Stopfen, die in die Füllöffnungen eingepresst werden. Die Füllöffnungen sind vorzugsweise rund und erleichtert dadurch ein dichtes Verschließen. Die mindestens zwei Stopfen sind vorzugsweise durch ein gemeinsames Stopfenbauteil gebildet.

Es kann vorgesehen sein, die Füllöffnungen bereits in einem Zustand vor Befüllung mittels eines temporären Stopfens verschlossen zu halten. Ein solcher temporärer Stopfen kann einen Innenraum des Flüssigkeitsspeichers während der Handhabung steril halten. Die temporären Stopfen können von außen in den jeweiligen Flüssigkeitsspeicher eindrückbar sein, um anschließend Befüllen zu können.

Vorzugweise sind die temporären Stopfen einstückig ausgebildet und insbesondere vorzugsweise mittels eines Filmscharniers an einer umgebenden Wandung der jeweiligen Füllöffnung angebunden. Alternativ können die Füllöffnungen vor Befüllung von einer dünnen und ggf. mit der umgebenden Wandung einstückige Membran verschlossen sein, die vor oder bei Einführen der Füllstutzen durchbrochen wird.

Bei bevorzugten Gestaltungen des Spenders mit zwei Flüssigkeitsspeichern sind diese zumindest in einem oberen Bereich nebeneinander liegend angeordnet, also so, dass bezogen auf den Querschnitt keiner der beiden Flüssigkeitsspeicher den anderen vollständig umgibt.

Es kann jedoch vorgesehen sein, dass zumindest in einem Bodenbereich des Flüssigkeitsspeichers der zweite Flüssigkeitsspeicher den ersten Flüssigkeitsspeicher umgibt, also umlaufend allseitig des inneren ersten Flüssigkeitsspeichers vorgesehen ist. Die Trennwandung zwischen den Flüssigkeitsspeichern ist im Bodenbereich dann als ringförmige Trennwandung ausgebildet, die eine erste Füllöffnung des ersten Flüssigkeitsspeichers umgibt und die von einer zweiten Füllöffnung des zweiten Flüssigkeitsspeichers umgeben ist. Diese konzentrische Anordnung der Füllöffnungen lässt eine drehstellungsunabhängige Befüllung der Flüssigkeitsspeicher zu.

Die beschriebene konzentrische Anordnung der Flüssigkeitsspeicher ist vorzugsweise auf den Bodenbereich beschränkt, wobei in einem oberen Bereich die Flüssigkeitsspeicher in der beschriebenen Weise nebeneinander angeordnet sind. In einem Übergangsbereich ist eine Überführungswandungsstruktur vorgesehen, die die nicht-ringförmige Trennwandung im oberen Bereich in die ringförmige Trennwandung im Bodenbereich überführt.

Um zu gewährleisten, dass das Ansaugrohr im Bodenbereich eine für den Füllvorgang unproblematische Position einnimmt, kann auch vorgesehen sein, dass ein unterer Teilabschnitt des Ansaugrohres fest mit einem Speicherbodenbauteil verbunden ist. Das Ansaugrohr oder ein Teilstück dessen ist in diesem Falle vor der Montage des Speicherbodenbauteils bereits an diesem befestigt oder einstückiger Teil dessen.

Das Ansaugrohr wird während der Montage an die Ansaugöffnung oder einen dortigen Teil des Ansaugrohres angeschlossen. Vorzugsweise sind am Ansaugrohr bodenseitige Führungsflächen, beispielsweise mit einer inneren oder äußeren Trichterstruktur, zum geführten Ansetzen des Ansaugrohres an die Ansaugöffnung vorgesehen. Die Führungsflächen sind vorzugsweise an einer Mehrzahl von Einzelrippen in Form von Einführungsschrägen vorgesehen.

Die bis hier beschriebene Bauweise der Flüssigkeitsspeicher ist insbesondere für Spender mit zwei Flüssigkeitsspeichern vorgesehen. Sie kann aber auch bei Spendern mit drei oder mehr Flüssigkeitsspeichern und vorzugsweise ebenso vielen Pumpeinrichtungen verwendet werden. Die genannten Erfindungsaspekte hinsichtlich der Bauweise der Flüssigkeitsspender erstrecken sich darüber hinaus auch auf Spender mit nur einem Flüssigkeitsspeicher.

Neben dem beschriebenen Spender umfasst die Erfindung auch ein Verfahren zur Herstellung und insbesondere zur Befüllung eines Flüssigkeitsspenders. Der Flüssigkeitsspender weist in der oben bereits beschriebenen Weise mindestens zwei Flüssigkeitsspeicher auf, zu denen jeweils eine Füllöffnungen im Bodenbereich der Flüssigkeitsspeicher vorgesehen ist. Vorzugsweise sind die Flüssigkeitsspeicher in oben beschriebener Art und Weise mit einer Gestaltung versehen, bei der das Ansaugrohr nicht in den Bereich der Füllöffnung ragt.

Das Befüllen der Flüssigkeitsspeicher erfolgt mit einer Ausrichtung des Flüssigkeitsspenders, bei der die Füllöffnungen nach oben weisen und dementsprechend eine Austragöffnung und/oder ein Betätigungsdrücker nach unten weisen. Ein Füllkopf mit zwei oder entsprechend mehr Füllstutzen ist vorgesehen, die gleichzeitig oder nacheinander in die Füllöffnungen des Spenders einfahren und dort die jeweilige Ausgangsflüssigkeit abgeben. Somit werden die Flüssigkeitsspeicher gefüllt. Anschließend werden die Flüssigkeitsspeicher mittels Stopfen oder mittels eines geschlossenen Speicherbodenbauteils verschlossen.

Vorzugsweise ist bereits während der Montage und vor dem vor dem Befüllen des Spenders ein Speicherbodenbauteil, in dem die mindestens eine Füllöffnung vorgesehen ist, an einem Hauptspeicherkörper angebracht worden, wobei im Zuge dieses Anbringens mindestens ein Ansaugrohr des Flüssigkeitsspeichers vom Speicherbodenbauteil von der mindestens einen Füllöffnung weggedrückt wurde.

Im Falle einer Gestaltung der Füllöffnung mit konzentrischer Anordnung erfolgt die Befüllung vorzugsweise mittels eines Füllkopfes, der über einen inneren Füllstutzen und einen äußeren Füllstutzen verfügt, wobei der innere Füllstützen innerhalb des äußeren Füllstutzens angeordnet ist. Vorzugsweise ist der innere Füllstutzen durch eine zweischalige Zwischenwandung von einem flüssigkeitsführenden Bereich des äußeren Füllstutzens getrennt. Eine äußere Schale der Zwischenwandung ist während der Befüllung außerhalb der ringförmigen Trennwandung der Flüssigkeitsspeicher angeordnet und eine innere Schale der Zwischenwandung ist während der Befüllung innerhalb dieser Trennwandung angeordnet.

Neben der Bauweise des Speicherbodens kann auch durch eine besondere Gestaltung der verwendeten Füllstutzen gewährleistet werden, dass ein Ansaugrohr den Füllvorgang nicht stört. Hierfür ist vorgesehen, dass die Flüssigkeit durch mindestens eine seitlich am Füllstutzen vorgesehene laterale Austrittsöffnung abgegeben wird. Vorzugsweise wird durch die Form des Füllstutzens mit einer Verdrängergeometrie an seiner distalen Stirnfläche bewirkt, dass das Ansaugrohr beim Einfahren in die Füllöffnung an der Stirnfläche abgleitet, insbesondere vorzugsweise weg von der lateralen Austrittsöffnung.

Im befüllten Zustand sind die Flüssigkeitsspeicher mitAusgangsflüssigkeiten befüllt, die in vermengter Form eine kosmetische oder pharmazeutische Flüssigkeit bilden. Insbesondere kann es sich um eine Formulierung handeln, die Stickoxid (NO) enthält.

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt ein erstes Ausführungsbeispiel eines erfindungsgemäßen Flüssigkeitsspenders in einer Gesamtdarstellung.
Fig. 2A-2C zeigen den Flüssigkeitsspender der Fig. 1 während der Befüllung.
Fig. 3 zeigt einen Flüssigkeitsspender gemäß einem zweiten Beispiel, das nicht den Ansprüchen entspricht.
Fig. 4A bis 4C zeigen ein drittes Beispiel, das nicht den Ansprüchen entspricht, in einer Gesamtdarstellung sowie den Ablauf bei der Befüllung.
Fig. 5A bis 5D zeigen die Montage und Befüllung eines Flüssigkeitsspenders gemäß einem vierten Ausführungsbeispiel der Erfindung.
Fig. 6A bis 6D zeigen einen Flüssigkeitsspender gemäß einem Beispiel, das nicht den Ansprüchen entspricht.
Fig. 7A bis 7D zeigen einen Flüssigkeitsspender gemäß einem sechsten Beispiel, das nicht den Ansprüchen entspricht.
Fig. 8A bis 8D zeigen den Befüllvorgang eines Spenders mit zwei Flüssigkeitsspeichern mitsamt eines besonders gestalteten Füllkopfs.

### DETAILLIERTE BESCHREIBUNG DER AUSFÜHRUNGSBEISPIELE

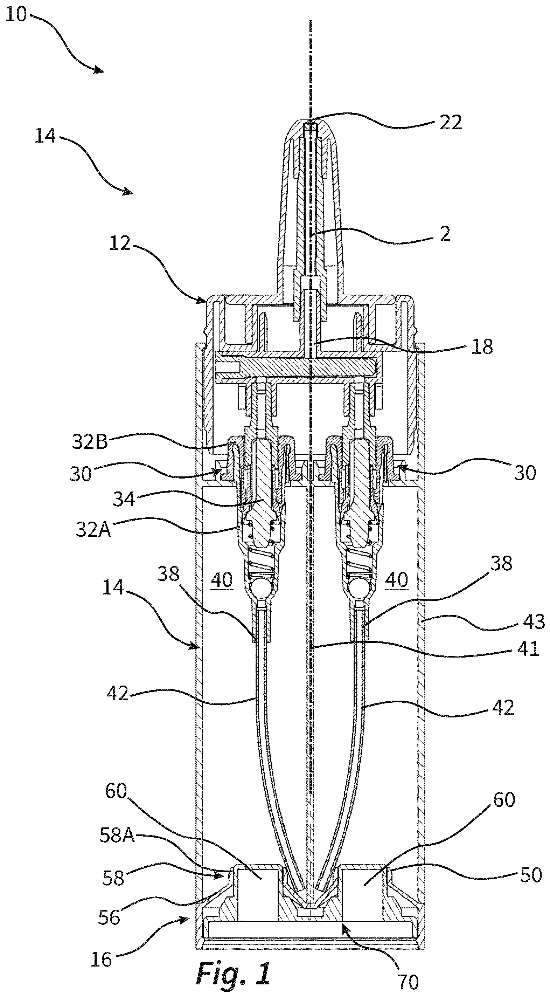

Die Fig. 1 zeigt einen Flüssigkeitsspender 10, der zur Abgabe einer Flüssigkeit ausgebildet ist, die aus zwei Ausgangsflüssigkeiten im Zuge des Austrages durch Vermengung erzeugt wird. Der Flüssigkeitsspender 10 ist entlang einer Haupterstreckungsrichtung 2 ausgerichtet und verfügt an seinem oberen Ende über eine Austragöffnung 22, durch die hindurch Flüssigkeit abgegeben werden kann. Diese Flüssigkeit wird durch einen Austragkanal 18 der Austragöffnung 22 zugeführt. Der Austragkanal 18 wird durch zwei Pumpeinrichtungen 30 gespeist.

Zur Betätigung der Pumpeinrichtungen 30 weist der Flüssigkeitsspender einen Betätigungsdrücker 12 auf, der auf Kolbenbauteile 34 beider Pumpeinrichtungen 30 wirkt und diese daher gleichzeitig betätigt. Hierdurch wird Flüssigkeit aus den Pumpkammern der Pumpeinrichtungen in Richtung der Austragöffnung 22 gedrückt.

Die Pumpeinrichtungen 30 sind dafür ausgebildet, Flüssigkeit aus zwei längserstreckten Flüssigkeitsspeichern 40 zu fördern. Sie sind jeweils an einer den Flüssigkeitsspeicher 40 nach oben abschließenden Zwischenwandung mittels eines oberen Pumpengehäuseteils 32B befestigt. Die Pumpeinrichtungen 30 erstrecken sich mit einem unteren Pumpengehäusebauteil 32A bis in die Flüssigkeitsspeicher 40. Am unteren Ende der Pumpeinrichtungen 30 sind jeweils Ansaugöffnungen 38 vorgesehen, wobei an diesen Ansaugöffnungen 38 jeweils ein Ansaugrohr 42 angebracht ist, welches sich bis in einen Bodenbereich der Flüssigkeitsspeicher 40 erstreckt. Diese Ansaugrohre 42 dienen dem Zweck, Flüssigkeit vom unteren Ende der Flüssigkeitsspeicher anzusaugen, so dass der Spender 10 in der in Fig. 1 dargestellten Ausrichtung verwendet und Flüssigkeit ausgetragen werden kann.

Die Flüssigkeitsspeicher 40 sind primär durch einen Hauptspeicherkörper 14 begrenzt, der sowohl eine Außenwand 43 als auch eine Trennwandung 41 zum überwiegenden Teil bildet. Am unteren Ende des Flüssigkeitsspenders 10 ist zum Verschließen des Hauptspeicherkörpers 14 ein Speicherboden 50 vorgesehen, wobei dieser vorliegend durch ein getrennt hergestelltes Speicherbodenbauteil 16 gebildet wird, welches beispielsweise mittels einer Schweißverbindung, einer Pressverbindung oder einer Klebeverbindung mit dem Hauptspeicherkörper 14 verbunden ist.

Der Speicherboden 50 weist zwei Füllöffnungen 60, die im Zustand der Fig. 1 bereits mittels eines Stopfenbauteils 70 verschlossen sind, auf. Weiterhin weist der Speicherboden 50 eine Haltegeometrie 58, insbesondere gebildet durch Haltewandungen 58A, auf, die sich so weit nach oben erstrecken, dass die Ansaugrohre 42 hierdurch von den Füllöffnungen 60 ferngehalten werden können. In Fig. 1 ist gut zu erkennen, dass die Ansaugrohre 42 durch die Haltewandungen 58A von den Füllöffnungen 60 weggedrückt werden. Durch schräg gestellte Leitflächen 56 am Speicherboden 50 wird gewährleistet, dass die Ansaugrohre 42 bei der Montage von den Füllöffnungen 60 weggedrückt werden.

Diese Gestaltung des Speicherbodens, die in der Lage ist, die Position der unteren Enden der Ansaugrohre 42 zu gewährleisten, dient dem Zweck, die Befüllung des Flüssigkeitsspenders 10 zu erleichtern.

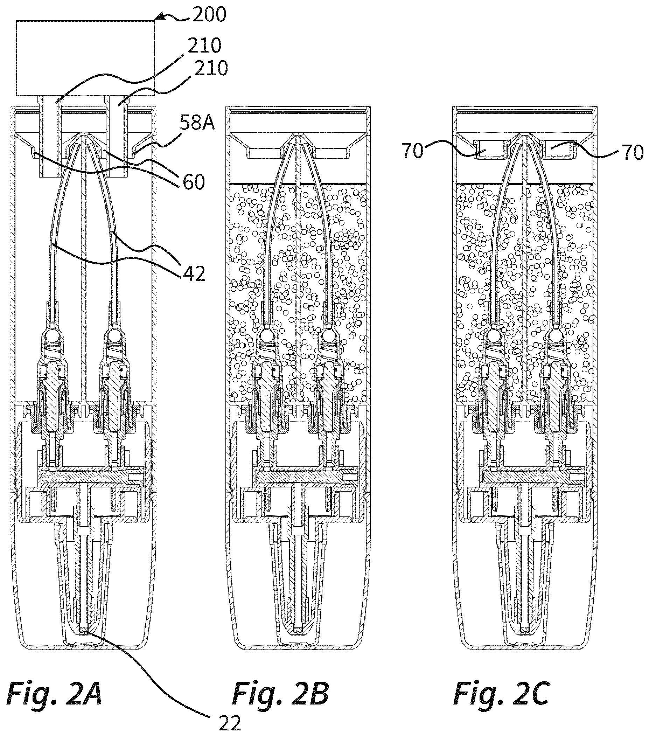

Der Füllvorgang ist in den Fig. 2A bis 2C verdeutlicht. Wie sich aus Fig. 2A ergibt, erfolgt der Füllvorgang bei einem erfindungsgemäßen Flüssigkeitsspender 10 in einer Lage, in der die Austragöffnung 22 nach unten und die bodenseitigen Füllöffnungen 60 nach oben weisen. Fig. 2A zeigt den Flüssigkeitsspender 10 in dieser Ausrichtung mit bereits eingefahrenen Füllstutzen 210, die Teil eines gemeinsamen Füllkopfes 200 sind. Die Füllstutzen 210 ragen durch die Füllöffnungen 60 bis in die Flüssigkeitsspeicher 40 hinein, wobei die Lagefixierung der Ansaugrohre 42 mittels der Haltewandungen 58A dazu führt, dass diese den Füllvorgang nicht stören.

Fig. 2B zeigt den befüllten Zustand der Flüssigkeitsspeicher 40. Ist dieser erreicht, so wird der Füllkopf 200 zurückgefahren und es werden Stopfenbauteile 70 in die Füllöffnungen 60 hineingedrückt. Vorzugsweise sind die Füllöffnungen 60 kreisrund, so dass eine zuverlässige Abdichtung durch Einpressen der Stopfenbauteile 70 erzielt werden kann. Es wird als vorteilhaft angesehen, wenn das Verschließen der Flüssigkeitsspeicher nach der Befüllung mit einfachen Montagetechniken möglich ist, insbesondere ohne Verkleben oder Verschweißen von Komponenten. Dies reduziert den Aufwand an der Abfüllstelle zum Verschließen der Flüssigkeitsspeicher 40.

Im Zustand der Fig. 2C ist der Flüssigkeitsspender 10 fertiggestellt und befüllt und somit in einem verkaufsfertigen Zustand.

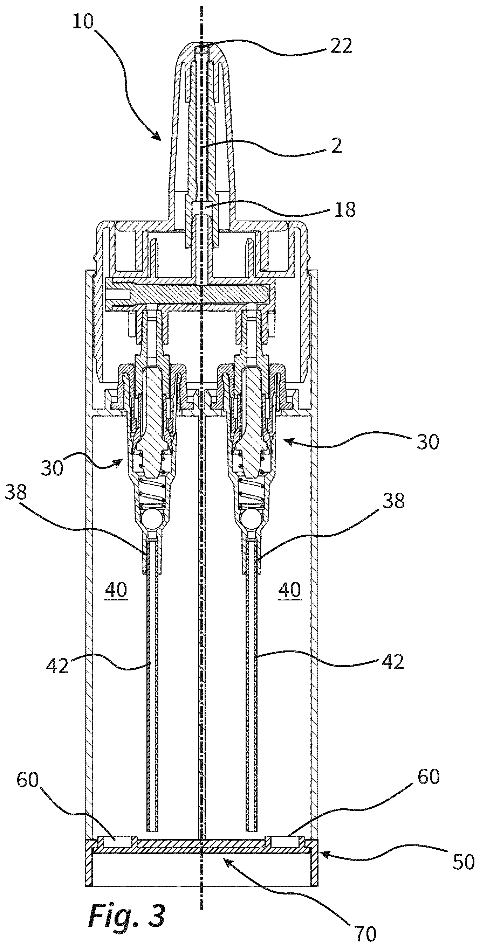

Die Fig. 3 zeigt eine andere Gestaltung eines Flüssigkeitsspenders 10. Wie auch bei den weiteren noch beschriebenen Gestaltungen der Flüssigkeitsspender 10 ist der Austragmechanismus mit den beiden Pumpeinrichtungen 30 und dem Austragkanal 18, der zur Austragöffnung 22 führt, jeweils unverändert. Bei der Gestaltung der Fig. 3 ist abweichend von der Gestaltung der Fig. 1 und 2 nicht vorgesehen, dass der Speicherboden 50 in mechanischer Art und Weise die Ansaugrohre 42 von den Füllöffnungen 60 fernhält. Stattdessen ist vorgesehen, dass die Füllöffnungen 60 bezogen auf die Haupterstreckungsrichtung 2 versetzt zu den Ansaugöffnungen 38 der Pumpeinrichtungen 30 angeordnet sind. Wenn Ansaugrohre einer ausreichenden Eigenstabilität verwendet werden, so lässt sich auch auf diesem Wege gewährleisten, dass die Füllstutzen 210 beim Befüllen der Flüssigkeitsspeicher 40 nicht mit den Ansaugrohren 42 kollidieren.

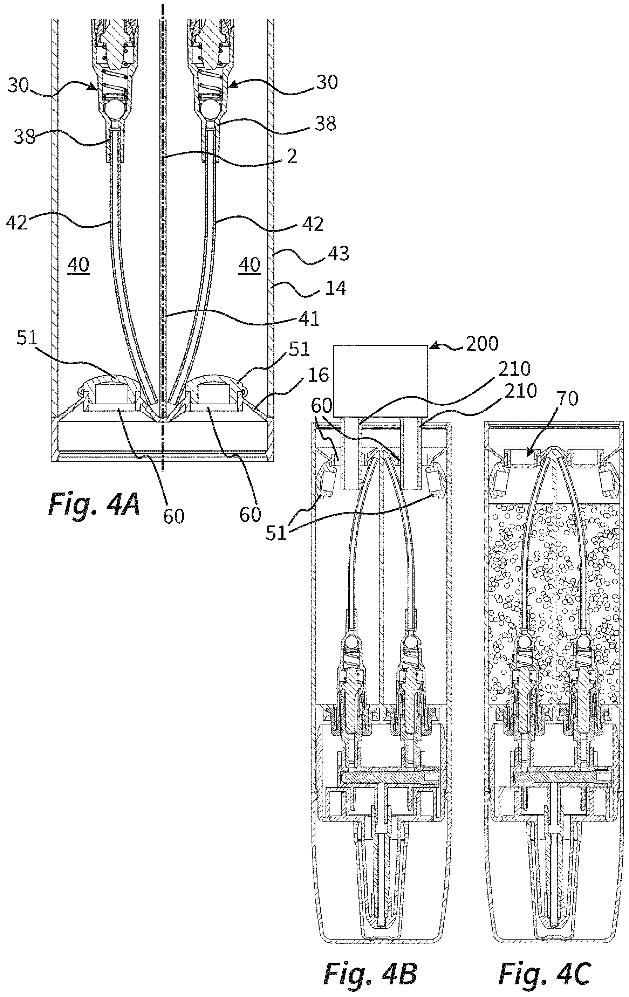

Die Gestaltung der Fig. 4A bis 4C ist wiederum derjenigen der Fig. 1 bis 2C recht ähnlich. Eine Besonderheit besteht hier darin, dass die Füllöffnungen 60 vor Befüllung der Flüssigkeitsspeicher 40 mit temporären Stopfen 51 verschlossen sind. Diese sind im Beispiel der Fig. 4A bis 4C, das nicht den Ansprüchen entspricht, mittels eines Filmscharniers mit dem Speicherbodenbauteil 16 verbunden.

Die Stopfen 51 bewirken, dass der Innenraum der Flüssigkeitsspeicher 40 während der Handhabung der noch leeren Flüssigkeitsspender 10 gegenüber einer Umgebung isoliert ist und somit steril gehalten werden kann.

Erst wenn in der in Fig. 4B verdeutlichten Weise bei der Befüllung der Füllkopf 200 mit den Füllstutzen 210 in die Füllöffnungen 60 einfährt, werden dabei die temporären Stopfen 51 aus den Füllöffnungen 60 herausgedrückt und anschließend die Befüllung begonnen.

In der in Fig. 4C verdeutlichten Weise werden nach Abschluss des Befüllens andere Stopfenbauteile 70 in die Füllöffnungen eingesetzt. Auch wenn in der Figur zwei separate Stopfenbauteile 70 Verwendung finden, so ist es auch möglich, dass entsprechend der Fig. 1 ein gemeinsames Stopfenbauteil 70 beide Füllöffnungen 60 verschließt.

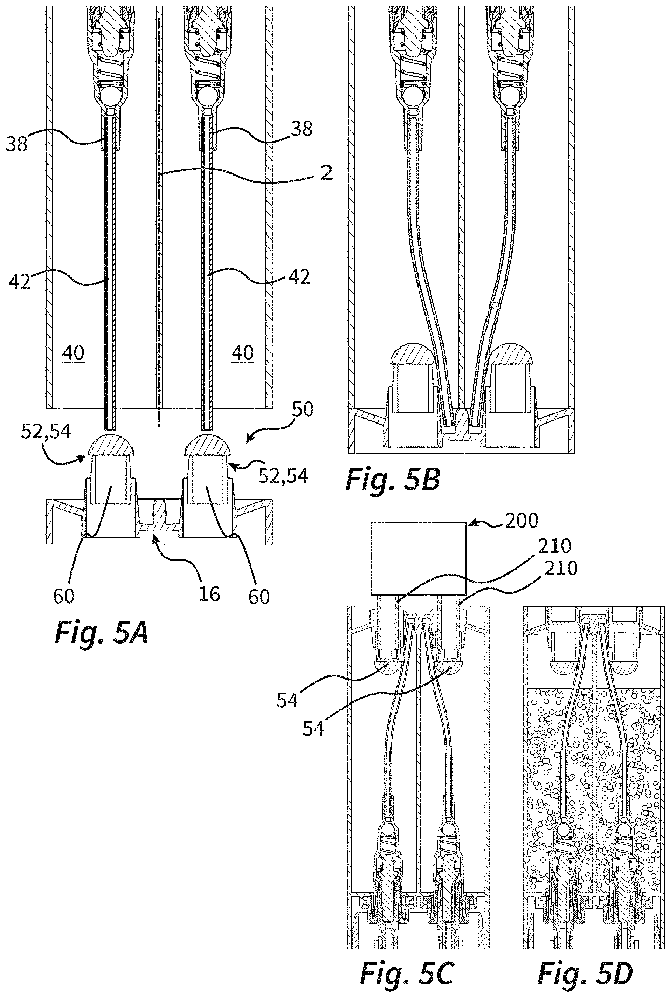

Die Fig. 5A bis 5D zeigen eine weitere Bauweise eines Spenders 10. Hier ist vorgesehen, dass der Speicherboden 50 im Bereich der Füllöffnungen 60 mit Sperrabschnitten 52 versehen ist, die die Füllöffnungen 60 überdecken und die an ihrer Oberseite eine sich verjüngende Formgebung aufweisen. Vorliegend sind die Sperrabschnitte 52 als separate Einsätze 54 vorgesehen, die insbesondere klemmend in die Füllöffnungen 60 des Speicherbodenbauteils 16 eingesetzt sind. Hier wäre alternativ jedoch auch eine einstückige Gestaltung möglich.

Fig. 5A verdeutlicht den Montagevorgang. Das Speicherbodenbauteil 16 mit den bereits eingesetzten Einsätzen 54 wird von unten an den Hauptspeicherkörper 14 mit den bereits montierten Pumpeinrichtungen 30 herangeführt. Die Ansaugrohre 42 geraten dabei in Kontakt mit den Sperrabschnitten 52 und werden von diesen zur Seite verdrängt, so dass sich schlussendlich die Anordnung der Ansaugrohre 42 entsprechend der Fig. 5B ergibt.

Alternativ kann auch vorgesehen sein, dass das Speicherbodenbauteil 16 vor der Montage der Pumpeinrichtungen 30 bereits am Hauptspeicherkörper 14 befestigt ist, beispielsweise durch Verpressen, Verschweißen oder Verkleben, und dass beim Einsetzen der Pumpeinrichtungen 30 mit den daran bereits angebrachten Ansaugrohren 42 die Sperrabschnitte 52 die Ansaugrohre entsprechend zur Seite drücken.

Die Fig. 5C und 5D verdeutlichen den Füllvorgang. Wie in Fig. 5C dargestellt ist, fahren wiederum zwei Füllstutzen eines Füllkopfes 200 in die Füllöffnungen 60 ein, wobei die hier angeordneten Einsätze 54 das Einfahren der Füllstutzen und das Einleiten der jeweiligen Flüssigkeiten nicht behindern.

Alternativ kann auch vorgesehen sein, dass die Füllstutzen die Einsätze 54 in die Flüssigkeitsspeicher 40 hineindrücken und damit vom Speicherbodenbauteil 16 trennen.

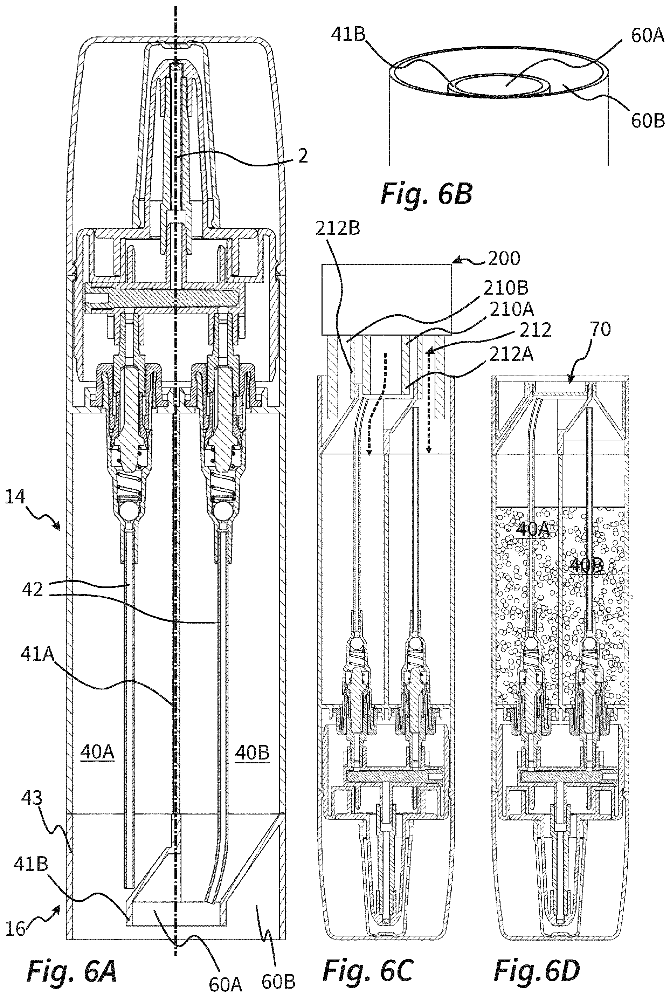

Bei der Gestaltung der Fig. 6A bis 6D weist der Flüssigkeitsspender 10 die Besonderheit auf, dass die Flüssigkeitsspeicher 40 in einem oberen Bereich nebeneinander angeordnet sind, so dass sie jeweils an einer Außenwand 43 des Hauptspeicherkörpers 14 anliegen. In einem unteren Bereich, in dem auch die Füllöffnungen 60A, 60B vorgesehen sind, ist die Konfiguration jedoch eine andere. Hier umgibt der Flüssigkeitsspeicher 40A den Flüssigkeitsspeicher 40B. Die Trennwandung 41A, die im oberen Teil zentrisch den Querschnitt des Hauptspeicherkörpers 14 in zwei Bereiche untergliedert, geht im unteren Bereich des Flüssigkeitsspenders 10 in eine ringförmige Trennwandung 41B über. Wie aus Fig. 6A und insbesondere aus Fig. 6B zu ersehen ist, führt dies dazu, dass sich konzentrische Füllöffnungen 60A und 60B ergeben.

Die Fig. 6C zeigt den Füllvorgang bei einer solchen Konfiguration. Es findet ein Füllkopf 200 Verwendung, der einen inneren Füllstutzen 210A und einen äußeren Füllstutzen 210B aufweist. Eine Zwischenwandung 212 zwischen den jeweils flüssigkeitsführenden Bereichen verfügt über eine innere Schale 212A und eine äußere Schale 212B, wobei während der Befüllung die innere Schale 212A innerhalb der Trennwandung 41B angeordnet ist und die äußere Schale 212B außerhalb der Trennwandung 41B.

Ein wesentlicher Vorteil, der sich hieraus ergibt, liegt darin, dass die Drehstellung des Flüssigkeitsspenders 10 während der Befüllung keine Relevanz hat. Ein aufwendiger Ausrichtprozess entfällt daher.

Wie in Fig. 6D verdeutlicht, werden die Flüssigkeitsspeicher 40A, 40B nach Befüllung mittels eines gemeinsamen Stopfenbauteils 70 verschlossen, welches eine an die Anordnung der Füllöffnungen 60A, 60B angepasste Formgebung aufweist.

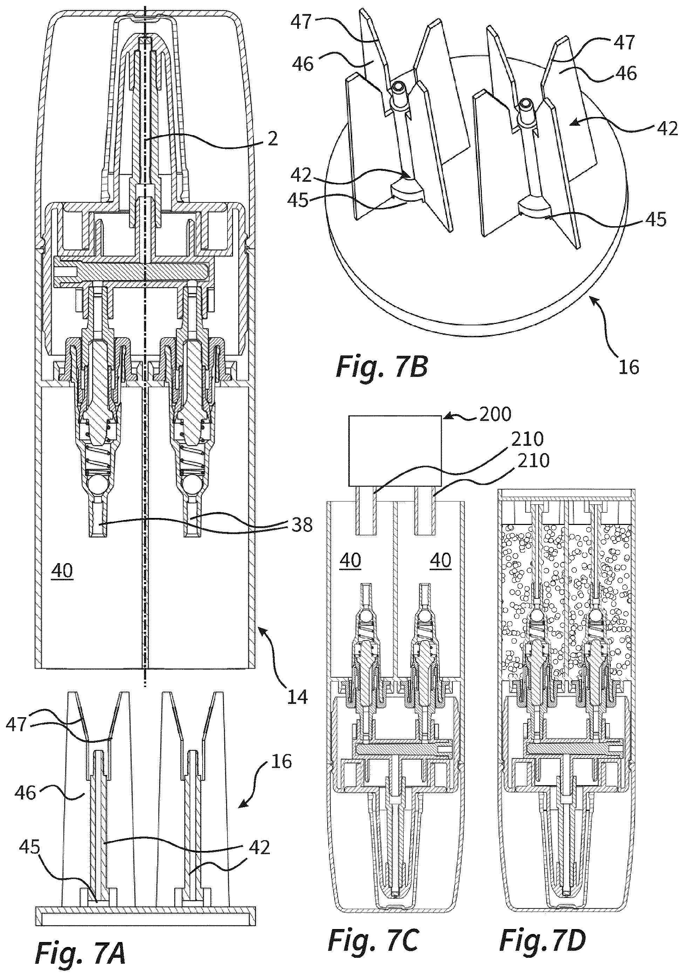

Bei der Gestaltung der Fig. 7A bis 7D ist ein nochmals deutlich verändertes Konzept vorgesehen, das nicht den Ansprüchen entspricht. Hier ist vorgesehen, dass das Speicherbodenbauteil 16 bereits vor der Anbringung am Hauptspeicherkörper 14 mit den Ansaugrohren 42 versehen ist. Wie aus Fig. 7A und 7B ersichtlich, sind diese Ansaugrohre fest und vorzugsweise einstückig Teil des Speicherbodenbauteils 16. An ihrem unteren Ende weisen sie laterale Auslässe 45 auf. Zur Erzielung von Stabilität und zur vereinfachten Montage sind sie jeweils mit vier umlaufend verteilten Einzelrippen 46 versehen, an denen trichterförmig ausgerichtete Führungsflächen 47 vorgesehen sind.

Wie Fig. 7C zeigt, sind die Ansaugrohre 42 somit zum Zeitpunkt der Befüllung der Flüssigkeitsspeicher 40 noch nicht montiert. Zunächst werden die Flüssigkeitsspeicher 40 in der in Fig. 7C verdeutlichten Weise mit Flüssigkeit befüllt. Erst anschließend wird das Speicherbodenbauteil 16 mit den Ansaugrohren 42 mit dem Hauptspeicherkörper 14 verbunden, beispielsweise mittels einer Pressverbindung, einer Klebeverbindung oder einer Schweißverbindung.

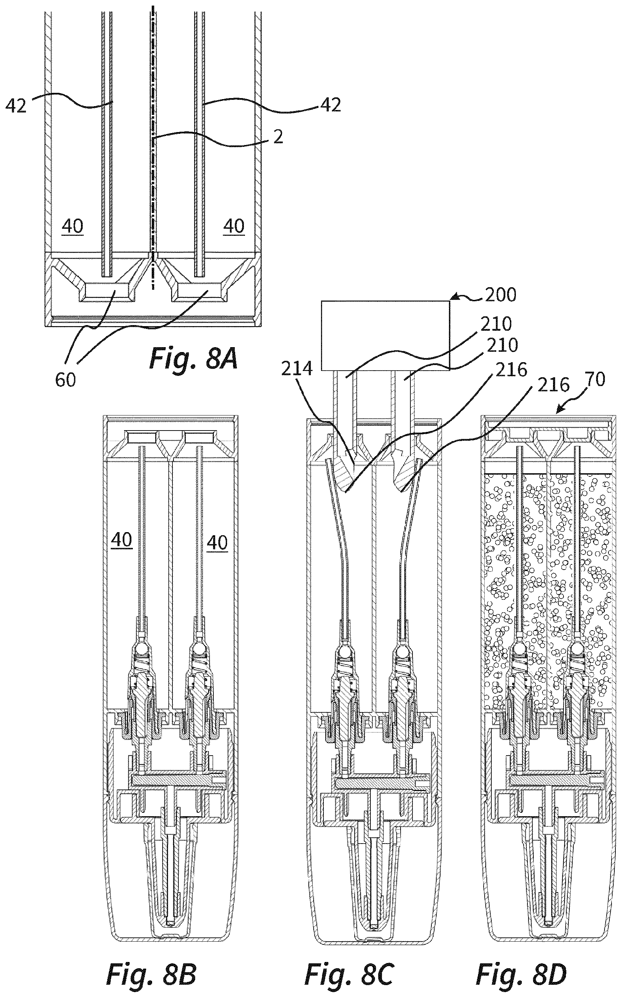

Die Fig. 8A bis 8D verdeutlichen eine weitere Möglichkeit einer vorteilhaften Befüllung der Flüssigkeitsspeicher 40. Bei dieser Variante ist, wie in Fig. 8A verdeutlicht, zunächst keinerlei Gewähr getroffen, dass die Ansaugrohre 42 von den Füllöffnungen 60 ferngehalten werden.

Allerdings finden hier besondere Füllstutzen 210 Verwendung. Diese beiden Füllstutzen 210 sind an ihrem distalen Ende mit einer Verdrängergeometrie 216 versehen, vorliegend mit einer sich bezogen auf Fig. 8C nach unten verjüngenden Spitze. Werden diese Füllstutzen 210 in die Füllöffnungen 60 eingefahren, so drücken sie währenddessen die Ansaugrohre 42 zur Seite. Die Befüllung der Flüssigkeitsspeicher 40 erfolgt dann über seitlich an den Füllstutzen 210 vorgesehene Austrittsöffnungen 214. Sobald die Befüllung abgeschlossen ist, werden die Füllöffnungen in der bereits beschriebenen Weise über ein gemeinsames Stopfenbauteil 70 verschlossen.

## Patentansprüche

1. Flüssigkeitsspender (10) zur Abgabe einer kosmetischen oder pharmazeutischen Flüssigkeit, die durch Vermengung mindestens zweier Ausgangsflüssigkeiten erzeugt wird, mit den folgenden Merkmalen:
a. der Flüssigkeitsspender (10) weist mindestens zwei Flüssigkeitsspeicher (40) zur Aufnahme der Ausgangsflüssigkeiten auf, wobei die Flüssigkeitsspeicher (40) sich in einer Haupterstreckungsrichtung (2) des Flüssigkeitsspenders (10) erstrecken und voneinander durch eine Trennwandung (41) getrennt sind, und
b. der Flüssigkeitsspender (10) weist mindestens eine Austragöffnung (22) sowie mindestens eine Pumpeinrichtung (30) auf, mittels derer Flüssigkeit aus den Flüssigkeitsspeichern (40) zur Austragöffnung (22) gefördert werden kann, und
c. an einem oberen Ende der Flüssigkeitsspeicher (40) ist jeweils eine Ansaugöffnung (38) zur Entnahme der Flüssigkeit mittels der mindestens einen Pumpeinrichtung (30) vorgesehen, und
d. von der Ansaugöffnung (38) erstreckt sich jeweils ein Ansaugrohr (42) zu einem unteren Ende des jeweiligen Flüssigkeitsspeichers (40), und
e. in einem Bodenbereich am unteren Ende der Flüssigkeitsspeicher (40) ist jeweils eine Füllöffnung (60) vorgesehen,
**gekennzeichnet durch** eines der folgenden Merkmale:
f. die Flüssigkeitsspeicher weisen im Bodenbereich einen Speicherboden (50) auf, der von jeweils einer Füllöffnung (60) durchdrungen ist, wobei die Speicherböden (50) jeweils zumindest abschnittsweise mit schräggestellten Leitflächen (56) versehen sind, durch die Ansaugrohre (42) während der Montage des Flüssigkeitsspenders (10) von der jeweiligen Füllöffnung (60) weggedrückt werden, oder
g. im Bodenbereich der Flüssigkeitsspeicher (40) sind Sperrabschnitte (52) vorgesehen, die innerhalb des jeweiligen Flüssigkeitsspeichers (40) fluchtend mit den jeweiligen Füllöffnungen (60) angeordnet sind und die während der Montage des Flüssigkeitsspenders (10) ein Einfahren des jeweiligen Ansaugrohres (42) in die Füllöffnungen (60) blockieren.

2. Flüssigkeitsspender (10) nach Anspruch 1 mit dem folgenden weiteren Merkmal:
a. die Speicherböden (50) der zwei Flüssigkeitsspeicher (40) sind durch ein gemeinsames einstückiges Speicherbodenbauteil (16) gebildet, und/oder
b. die Speicherböden (50) sind durch mindestens ein Speicherbodenbauteil (16) gebildet, welches von einem Hauptspeicherkörper (14) getrenntes Bauteil ausgebildet ist, wobei der Hauptspeicherkörper (14) vorzugsweise zwei kreisrunde Öffnungen aufweist, in die das mindestens eine Speicherbodenbauteil (16) eingesetzt ist, und/oder
c. das Speicherbodenbauteil (16) ist mit dem Hauptspeicherkörper (14) durch Verkleben, Verschweißen oder mittels einer Pressverbindung verbunden ist.

3. Flüssigkeitsspender (10) nach Anspruch 1 oder 2 mit dem folgenden weiteren Merkmal:
a. die Speicherböden (50) weisen jeweils mindestens eine Haltegeometrie (58) auf, durch die ein unteres Ende des jeweiligen Ansaugrohrs (42) von der jeweiligen Füllöffnung (60) ferngehalten wird.

4. Flüssigkeitsspender (10) nach Anspruch 3 mit dem folgenden weiteren Merkmal:
a. die Haltegeometrien (58) weisen jeweils eine die jeweilige Füllöffnung (60) umgebenden Haltewandung (58A) auf.

5. Flüssigkeitsspender (10) nach einem der vorstehenden Ansprüche, wobei der Flüssigkeitsspender das Merkmal g aus Anspruch 1 aufweist, mit mindestens einem der folgenden zusätzlichen Merkmale:
a. die Sperrabschnitte (52) werden durch von einem Speicherbodenbauteil (16) getrennte Einsätze (54) gebildet, die am Speicherbodenbauteil (16) befestigt sind, insbesondere in den Füllöffnungen (60) und insbesondere mittels einer Klemm- oder Rastverbindung, und/oder
b. die Sperrabschnitte (52) verfügen an ihrer nach oben weisenden Seite über eine von der ebenen Formgebung abweichende Formgebung, insbesondere über eine sich nach oben verjüngende Formgebung, und/oder
c. die Sperrabschnitte (52) verfügen an ihrer nach oben weisenden Seite über eine Formgebung in Art einer gegenüber der Haupterstreckungsrichtung (2) schräggestellten Ebene.

6. Flüssigkeitsspender (10) nach einem der vorstehenden Ansprüche mit dem folgenden weiteren Merkmal:
a. in einem Zustand vor Befüllung sind die Füllöffnungen (60) jeweils mittels eines Stopfens (51) temporär verschlossen,
vorzugsweise mit mindestens einem der folgenden zusätzlichen Merkmale:
b. die Stopfen (51) sind von außen in den jeweiligen Flüssigkeitsspeicher (40) eindrückbar und/oder
c. die Stopfen (51) sind einstückig mit dem mindestens einen Speicherbodenbauteil (16) ausgebildet und insbesondere vorzugsweise mittels eines Filmscharniers an einer umgebenden Wandung der jeweiligen Füllöffnung (60) angebunden.

7. Flüssigkeitsspender (10) nach einem der vorstehenden Ansprüche mit mindestens einem der folgenden weiteren Merkmale:
a. die Ansaugöffnungen (38) sind gegenüber den Füllöffnungen (60) bezogen auf die Haupterstreckungsrichtung (2) versetzt angeordnet, und
b. die Ansaugrohre (42) sind im Wesentlichen geradlinig ausgebildet, so dass sie entsprechend der Ansaugöffnung (38) gegenüber den Füllöffnungen versetzt sind.

8. Flüssigkeitsspender (10) nach einem der vorstehenden Ansprüche mit den folgenden weiteren Merkmalen:
a. zumindest in einem der mindestens einen Pumpeinrichtung (30) gegenüberliegenden Bodenbereich des Flüssigkeitsspeichers umgibt der zweite Flüssigkeitsspeicher (40) den ersten Flüssigkeitsspeicher (40), und
b. die Trennwandung (41) ist im Bodenbereich als ringförmige Trennwandung (41B) ausgebildet, die eine erste Füllöffnung (60A) des ersten Flüssigkeitsspeichers (40) umgibt und die von einer zweiten Füllöffnung (60B) des zweiten Flüssigkeitsspeichers (40) umgeben ist.

9. Flüssigkeitsspender (10) nach Anspruch 8 mit den folgenden weiteren Merkmalen:
a. zumindest in einem oberen Bereich der Flüssigkeitsspeicher (40) sind die Flüssigkeitsspeicher (40) nebeneinander angeordnet, so dass beide Flüssigkeitsspeicher jeweils an eine gemeinsame Außenwand (43) sowie an eine von der Außenwand (43) ausgehende nicht-ringförmige Trennwandung (41A) angrenzen, und
b. zwischen dem Bodenbereich und dem oberen Bereich ist eine Überführungswandungsstruktur vorgesehen, die die nicht-ringförmige Trennwandung (41A) in die ringförmige Trennwandung (41B) überführt.

10. Flüssigkeitsspender (10) nach einem der vorstehenden Ansprüche mit den folgenden weiteren Merkmalen:
a. zumindest ein unterer Teilabschnitt (44) des Ansaugrohres (42) ist fest mit einem Speicherbodenbauteil (16) verbunden,
vorzugsweise mit mindestens einem der folgenden zusätzlichen Merkmale:
b. der untere Teilabschnitt (44) des Ansaugrohres (42) ist einstückig mit dem Speicherbodenbauteil (16) verbunden.

11. Flüssigkeitsspender (10) nach Anspruch 10 mit dem folgenden weiteren Merkmal:
a. am unteren Teilabschnitt (44) des Ansaugrohres sind Führungsflächen (47) zum geführten Ansetzen des unteren Teilabschnitts (44) an die Ansaugöffnung (38) vorgesehen,
vorzugsweise mit mindestens einem der folgenden weiteren Merkmale:
b. die Führungsflächen (47) sind an einer Mehrzahl von Einzelrippen (46) in Form von Einführungsschrägen vorgesehen, und/oder
c. die Führungsflächen bilden eine Trichterstruktur.

12. Flüssigkeitsspender (10) nach einem der vorstehenden Ansprüche mit mindestens einem der folgenden weiteren Merkmale:
a. der Flüssigkeitsspender (10) weist einen Betätigungsdrücker (12) auf, der gegenüber den Flüssigkeitsspeichern (40) verlagerbar ist, wobei die mindestens eine Pumpeinrichtung (30) dafür ausgebildet ist, durch diese Verlagerung des Betätigungsdrückers (12) gegenüber den Flüssigkeitsspeichern (40) Flüssigkeit aus den Flüssigkeitsspeichern (40) zur Austragöffnung (22) zu fördern, und/oder
b. der Flüssigkeitsspender (10) weist zwei Pumpeinrichtungen (30) auf, die vorzugweise zur gleichzeitigen oder zur zumindest phasenweise zeitlich überlappenden Betätigung mechanisch miteinander verbunden sind, wobei insbesondere zwei Kolbenbauteile (34) der Pumpeinrichtungen (30) miteinander gekoppelt sind, und/oder
c. der Flüssigkeitsspender (10) weist einen Hauptspeicherkörper (14) auf, der einstückig zumindest abschnittsweise eine Außenwand (43) beider Flüssigkeitsspeicher (40) und zumindest abschnittsweise die die Flüssigkeitsspeicher (40) trennende Trennwandung (41) bildet, und/oder
d. in einem befüllten Zustand sind die Füllöffnungen (60) mittels mindestens eines Stopfenbauteils (70) verschlossen, vorzugsweise mittels eines gemeinsamen Stopfenbauteils (70) zum Verschließen beider Füllöffnungen (60), und/oder
e. der Flüssigkeitsspender (10) umfasst zwei Pumpeinrichtungen (30), die jeweils einem der beiden Flüssigkeitsspeicher (40) zugeordnet sind, wobei vorzugsweise an einem Ansaugstutzen der Pumpeinrichtungen (30) die jeweilige Ansaugöffnung (38) vorgesehen ist, an der das jeweiligen Ansaugrohr (42) angebracht ist, und/oder
f. mindestens ein Ansaugrohr (42) ist mittels eines von der Ansaugöffnung (38) beabstandeten Haltemittels zusätzlich gehalten, insbesondere im Bereich einer Mantelwandung des jeweiligen Flüssigkeitsspeichers, und/oder

13. Verfahren zum Herstellung und Befüllung eines Flüssigkeitsspenders (10) mit zwei Flüssigkeitsspeichern (40) mit den folgenden Merkmalen:
a. der Flüssigkeitsspender (10) weist zwei Füllöffnungen (60) auf, die jeweils in einem Bodenbereich der Flüssigkeitsspeicher (40) vorgesehen sind, und
b. das Befüllen der Flüssigkeitsspeicher (40) erfolgt mit einer Ausrichtung des Flüssigkeitsspenders (10), bei der die Füllöffnungen (60) nach oben weisen, und
c. das Befüllen erfolgt mittels mindestens eines Füllkopfes (200), der über mindestens zwei Füllstutzen (210) verfügt, die gleichzeitig oder nacheinander in die Füllöffnungen (60) einfahren und dort Flüssigkeit abgeben und somit die Flüssigkeitsspeicher (40) füllen,
**gekennzeichnet durch** das folgende zusätzliche Merkmal:
d. das Befüllen erfolgt nachdem zuvor mindestens ein Speicherbodenbauteil (16), in dem die mindestens eine Füllöffnung (60) vorgesehen ist, an einem Hauptspeicherkörper (14) angebracht wurde, wobei im Zuge der Anbringung mindestens ein Ansaugrohr (42) des Flüssigkeitsspeichers (40) mittels des Speicherbodenbauteils (16) von der mindestens einen Füllöffnung (60) weggedrückt worden ist.

14. Verfahren nach Anspruch 13 mit mindestens einem der folgenden zusätzlichen Merkmale:
a. zumindest in einem Bodenbereich der Flüssigkeitsspeicher (40A, 40B) umgibt der zweite Flüssigkeitsspeicher (40B) den ersten Flüssigkeitsspeicher (40A), wobei hierfür eine Trennwandung (41) zumindest abschnittsweise als ringförmige Trennwandung (41B) ausgebildet ist, die eine erste Füllöffnung (60A) des ersten Flüssigkeitsspeichers (40A) umgibt und die von einer zweiten Füllöffnung (60B) des zweiten Flüssigkeitsspeichers (40B) umgeben ist, und
b. das Befüllen erfolgt mittels eines Füllkopfes, der über einen inneren Füllstutzen (210A) und einen äußeren Füllstutzen (210B) verfügt, wobei der innere Füllstützen (210A) innerhalb des äußeren Füllstutzens (210B) angeordnet ist,
vorzugsweise mit dem folgenden zusätzlichen Merkmal:
c. der innere Füllstutzen (210A) ist durch eine zweischalige Zwischenwandung (212) von einem flüssigkeitsführenden Bereich des äußeren Füllstutzens (210B) getrennt, wobei eine äußere Schale (212B) der Zwischenwandung (212) während der Befüllung außerhalb der ringförmigen Trennwandung (41B) angeordnet ist und wobei eine innere Schale (212A) der Zwischenwandung (212) während der Befüllung innerhalb der ringförmigen Trennwandung (41) angeordnet ist.

15. Verfahren nach einem der Ansprüche 13 oder 14 mit mindestens einem der folgenden zusätzlichen Merkmale:
a. während der Befüllung wird die Flüssigkeit durch eine seitlich am Füllstutzen (210) vorgesehene Austrittsöffnung (214) abgegeben, und/oder
b. der Füllstützen (210) verfügt über eine Verdrängergeometrie (216) an seiner distalen Stirnfläche, an der das Ansaugrohr (42) beim Einfahren in die Füllöffnung (60) abgleitet.

## Claims

1. Liquid dispenser (10) for dispensing a cosmetic or pharmaceutical liquid which is produced by mixing at least two starting liquids, with the following features:
a. the liquid dispenser (10) has at least two liquid reservoirs (40) for holding the starting liquids, wherein the liquid reservoirs (40) extend in a main direction of extent (2) of the liquid dispenser (10) and are separated from each other by a partition wall (41), and
b. the liquid dispenser (10) has at least one discharge opening (22) and at least one pump device (30) by means of which liquid can be delivered from the liquid reservoirs (40) to the discharge opening (22), and
c. a respective intake opening (38) for removing the liquid by means of the at least one pump device (30) is provided at a top end of the liquid reservoirs (40), and
d. a respective intake tube (42) extends from the intake opening (38) to a bottom end of the respective liquid reservoir (40), and
e. a respective filling opening (60) is provided in a base region at the bottom end of the liquid reservoirs (40),
**characterized by** one of the following features:
f. the liquid reservoirs have a reservoir base (50) in the base region, which is perforated by a filling opening (60) in each case, wherein the reservoir bases (50) are each provided with oblique guide surfaces (56) at least in some places, by virtue of which intake tubes (42) are pushed away from the respective filling opening (60) during the installation of the liquid dispenser (10), or
g. barrier sections (52), which are arranged inside the respective liquid reservoir (40) so that they are flush with the respective filling openings (60) and which block the respective intake tube (42) from entering the filling openings (60) during the installation of the liquid dispenser (10), are provided in the base region of the liquid reservoirs (40).

2. Liquid dispenser (10) according to Claim 1 with the following further feature:
a. the reservoir bases (50) of the two liquid reservoirs (40) are formed by a common one-piece reservoir base component (16), and/or
b. the reservoir bases (50) are formed by at least one reservoir base component (16) which is formed by a component separate from a main reservoir body (14), wherein the main reservoir body (14) preferably has two circular openings into which the at least one reservoir base component (16) is inserted, and/or
c. the reservoir base component (16) is connected to the main reservoir body (14) by being adhesively bonded or welded or by means of a press-fit connection.

3. Liquid dispenser (10) according to Claim 1 or 2 with the following further feature:
a. the reservoir bases (50) each have at least one holding geometry (58) by means of which a bottom end of the respective intake tube (42) is kept away from the respective filling opening (60).

4. Liquid dispenser (10) according to Claim 3 with the following further feature:
a. the holding geometries (58) each have a supporting wall (58A) surrounding the respective filling opening (60).

5. Liquid dispenser (10) according to one of the preceding claims, wherein the liquid dispenser has the feature g from Claim 1, with at least one of the following additional features:
a. the barrier sections (52) are formed by inserts (54) which are separate from a reservoir base component (16) and are fastened to the reservoir base component (16), in particular in the filling openings (60) and in particular by means of a clamped or latched connection, and/or
b. the barrier sections (52) have, on their upward facing side, a shape which deviates from the plane shape, in particular an upward tapering shape, and/or
c. the barrier sections (52) have, on their upward facing side, a shape in the manner of a plane which is oblique relative to the main direction of extent (2).

6. Liquid dispenser (10) according to one of the preceding claims with the following further feature:
a. the filling openings (60) are each temporarily closed by means of a stopper (51) in a state before the filling,
preferably with at least one of the following additional features:
b. the stoppers (51) can be pushed into the respective liquid reservoir (40) from outside, and/or
c. the stoppers (51) are designed as a single piece with the at least one reservoir base component (16) and in particular are attached to a surrounding wall of the respective filling opening (60) preferably by means of a film hinge.

7. Liquid dispenser (10) according to one of the preceding claims with at least one of the following further features:
a. the intake openings (38) are arranged so that they are offset relative to the filling openings (60) with respect to the main direction of extent (2), and
b. the intake tubes (42) are designed so that they are essentially straight, such that they are offset relative to the filling openings, corresponding to the intake opening (38).

8. Liquid dispenser (10) according to one of the preceding claims with the following further features:
a. at least in a base region, situated opposite the at least one pump device (30), of the liquid reservoir, the second liquid reservoir (40) surrounds the first liquid reservoir (40), and
b. the partition wall (41) is formed in the base region as an annular partition wall (41B) which surrounds a first filling opening (60A) of the first liquid reservoir (40) and which is surrounded by a second filling opening (60B) of the second liquid reservoir (40).

9. Liquid dispenser (10) according to Claim 8 with the following further features:
a. at least in an upper region of the liquid reservoirs (40), the liquid reservoirs (40) are arranged next to each other such that the two liquid reservoirs in each case adjoin a common outer wall (43) and a non-annular partition wall (41A) originating from the outer wall (43), and
b. a transfer wall structure, which transfers the non-annular partition wall (41A) into the annular partition wall (41B), is provided between the base region and the upper region.

10. Liquid dispenser (10) according to one of the preceding claims with the following further features:
a. at least one lower subsection (44) of the intake tube (42) is firmly connected to a reservoir base component (16),
preferably with at least one of the following additional features:
b. the lower subsection (44) of the intake tube (42) is integrally connected to the reservoir base component (16).

11. Liquid dispenser (10) according to Claim 10 with the following further feature:
a. guide surfaces (47) for the guided placement of the lower subsection (44) onto the intake opening (38) are provided on the lower subsection (44) of the intake tube,
preferably with at least one of the following further features:
b. the guide surfaces (47) are provided on a plurality of individual ribs (46) in the form of lead-in slopes, and/or
c. the guide surfaces form a funnel structure.

12. Liquid dispenser (10) according to one of the preceding claims with at least one of the following further features:
a. the liquid dispenser (10) has a push actuator (12) which can be displaced relative to the liquid reservoirs (40), wherein the at least one pump device (30) is designed to deliver liquid from the liquid reservoirs (40) to the discharge opening (22) by virtue of this displacement of the push actuator (12) relative to the liquid reservoirs (40), and/or
b. the liquid dispenser (10) has two pump devices (30) which are preferably mechanically connected to each other for the purpose of simultaneous or at least at times overlapping activation, wherein in particular two piston components (34) of the pump devices (30) are coupled to each other and/or
c. the liquid dispenser (10) has a main reservoir body (14) which, in a single piece, forms at least partially an outer wall (43) of both liquid reservoirs (40) and at least partially the partition wall (41) separating the liquid reservoirs (40), and/or
d. in a filled state, the filling openings (60) are closed by means of at least one stopper component (70), preferably by means of a common stopper component (70) for closing both filling openings (60), and/or
e. the liquid dispenser (10) comprises two pump devices (30) which are each associated with one of the two liquid reservoirs (40), wherein the respective intake opening (38) to which the respective intake tube (42) is attached is preferably provided on an intake nozzle of the pump devices (30), and/or
f. at least one intake tube (42) is additionally supported by means of a supporting means spaced apart from the intake opening (38), in particular in the region of an outer wall of the respective liquid reservoir.

13. Method for producing and filling a liquid dispenser (10) with two liquid reservoirs (40) with the following features:
a. the liquid dispenser (10) has two filling openings (60) which are each provided in a base region of the liquid reservoirs (40), and
b. the liquid reservoirs (40) are filled with an orientation of the liquid dispenser (10) in which the filling openings (60) face upwards, and
c. the filling is effected by means of at least one filling head (200) which has at least two filling nozzles (210) which enter the filling openings (60) simultaneously or one after the other and dispense liquid there and hence fill the liquid reservoirs (40),
**characterized by** the following additional feature:
d. the filling is effected after at least one reservoir base component (16), in which the at least one filling opening (60) is provided, has been attached to a main reservoir body (14), wherein at least one intake tube (42) of the liquid reservoir (40) has been pushed away from the at least one filling opening (60) by means of the reservoir base component (16) in the course of the attachment.

14. Method according to Claim 13 with at least one of the following additional features:
a. the second liquid reservoir (40B) surrounds the first liquid reservoir (40A) at least in a base region of the liquid reservoirs (40A, 40B), wherein for this purpose a partition wall (41) is designed at least in some places as an annular partition wall (41B) which surrounds a first filling opening (60A) of the first liquid reservoir (40A) and which is surrounded by a second filling opening (60B) of the second liquid reservoir (40B), and
b. the filling is effected by means of a filling head which has an inner filling nozzle (210A) and an outer filling nozzle (210B), wherein the inner filling nozzle (210A) is arranged inside the outer filling nozzle (210B),
preferably with the following additional feature:
c. the inner filling nozzle (210A) is separated from a liquid-guidance region of the outer filling nozzle (210B) by a double-shell intermediate wall (212), wherein an outer shell (212B) of the intermediate wall (212) is arranged outside the annular partition wall (41B) during the filling and wherein an inner shell (212A) of the intermediate wall (212) is arranged inside the annular partition wall (41) during the filling.

15. Method according to either of Claims 13 or 14 with at least one of the following additional features:
a. during the filling the liquid is dispensed through an outlet opening (214) provided in the side of the filling nozzle (210), and/or
b. the filling nozzle (210) has a displacement geometry (216) at its far end face on which the intake tube (42) slides when it enters the filling opening (60).

## Revendications

1. Distributeur de liquide (10) destiné à distribuer un liquide cosmétique ou pharmaceutique, qui est produit en mélangeant au moins deux liquides de départ, avec les caractéristiques suivantes :
a. le distributeur de liquide (10) comporte au moins deux réservoirs de liquide (40) destinés à recevoir les liquides de départ, les réservoirs de liquide (40) s'étendant dans une direction d'extension principale (2) du distributeur de liquide (10) et étant séparés l'un de l'autre par une cloison de séparation (41), et
b. le distributeur de liquide (10) comporte au moins une ouverture de décharge (22) ainsi qu'au moins un dispositif de pompage (30), au moyen duquel du liquide provenant des réservoirs de liquide (40) peut être transporté vers l'ouverture de décharge (22), et
c. une ouverture d'aspiration (38) destinée à prélever le liquide au moyen de l'au moins un dispositif de pompage (30) est prévue respectivement sur une extrémité supérieure des réservoirs de liquide (40), et
d. un tuyau d'aspiration (42) s'étend respectivement depuis l'ouverture d'aspiration (38) vers une extrémité inférieure du réservoir de liquide (40) respectif, et
e. une ouverture de remplissage (60) est prévue dans une zone de fond sur l'extrémité inférieure des réservoirs de liquide (40) respectivement,
**caractérisé par** une des caractéristiques suivantes :
f. les réservoirs de liquide comportent dans la zone de fond un fond de réservoir (50), qui est traversé par respectivement une ouverture de remplissage (60), les fonds de réservoir (50) étant pourvus chacun au moins par endroits de surfaces d'acheminement (56) inclinées, par lesquelles des tuyaux d'aspiration (42) sont retirés par pression de l'ouverture de remplissage (60) respective au cours du montage du distributeur de liquide (10), ou
g. des sections de blocage (52), qui sont disposées de manière alignée avec les ouvertures de remplissage (60) respectives à l'intérieur du réservoir de liquide (40) respectif et qui empêchent que le tuyau d'aspiration (42) respectif ne rentre dans les ouvertures de remplissage (60) au cours du montage du distributeur de liquide (10), sont prévues dans la zone de fond des réservoirs de liquide (40).

2. Distributeur de liquide (10) selon la revendication 1, avec la caractéristique supplémentaire suivante :
a. les fonds de réservoir (50) des deux réservoirs de liquide (40) sont formés par un composant de fond de réservoir (16) d'un seul tenant commun, et/ou
b. les fonds de réservoir (50) sont formés par au moins un composant de fond de réservoir (16), lequel est réalisé comme un composant séparé d'un corps de réservoir principal (14), le corps de réservoir principal (14) comportant de préférence deux ouvertures rondes circulaires, dans lesquelles l'au moins un composant de fond de réservoir (16) est inséré, et/ou
c. le composant de fond de réservoir (16) est relié au corps de réservoir principal (14) par collage, soudage ou au moyen d'une liaison par pressage.

3. Distributeur de liquide (10) selon la revendication 1 ou 2, avec la caractéristique supplémentaire suivante :
a. les fonds de réservoir (50) présentent chacun au moins une géométrie de maintien (58), par laquelle une extrémité inférieure du tuyau d'aspiration (42) respectif est maintenue à l'écart de l'ouverture de remplissage (60) respective.

4. Distributeur de liquide (10) selon la revendication 3, avec la caractéristique supplémentaire suivante :
a. les géométries de maintien (58) comportent chacune une cloison de maintien (58A) entourant l'ouverture de remplissage (60) respective.

5. Distributeur de liquide (10) selon l'une des revendications précédentes, le distributeur de liquide présentant la caractéristique g de la revendication 1, avec au moins une des caractéristiques supplémentaires suivantes :
a. les sections de blocage (52) sont formées par des inserts (54) séparés d'un composant de fond de réservoir (16), qui sont fixés sur le composant de fond de réservoir (16), en particulier dans les ouvertures de remplissage (60) et en particulier au moyen d'une liaison par serrage ou par enclenchement, et/ou
b. les sections de blocage (52) disposent sur leur côté pointant vers le haut d'un façonnage qui diverge du façonnage plat, en particulier d'un façonnage se rétrécissant vers le haut, et/ou
c. les sections de blocage (52) disposent sur leur côté pointant vers le haut d'un façonnage à la manière d'un plan incliné par rapport à la direction d'extension principale (2).

6. Distributeur de liquide (10) selon l'une des revendications précédentes, avec la caractéristique supplémentaire suivante :
a. les ouvertures de remplissage (60) sont temporairement fermées au moyen d'un bouchon (51) dans un état avant le remplissage,
de préférence avec au moins une des caractéristiques supplémentaires suivantes :
b. les bouchons (51) peuvent être enfoncés depuis l'extérieur dans le réservoir de liquide (40) respectif, et/ou
c. les bouchons (51) sont réalisés d'un seul tenant avec l'au moins un composant de fond de réservoir (16) et sont attachés à une cloison qui les entoure de l'ouverture de remplissage (60) respective en particulier de préférence au moyen d'une charnière à film.

7. Distributeur de liquide (10) selon l'une des revendications précédentes, avec au moins une des caractéristiques supplémentaires suivantes :
a. les ouvertures d'aspiration (38) sont disposées de manière décalée par rapport à la direction d'extension principale (2) en vis-à-vis des ouvertures de remplissage (60), et
b. les tuyaux d'aspiration (42) sont réalisés de manière sensiblement rectiligne de telle sorte qu'ils sont décalés en vis-à-vis des ouvertures de remplissage de manière à correspondre à l'ouverture d'aspiration (38).

8. Distributeur de liquide (10) selon l'une des revendications précédentes, avec les caractéristiques supplémentaires suivantes :
a. le deuxième réservoir de liquide (40) entoure le premier réservoir de liquide (40) au moins dans une zone de fond du réservoir de liquide faisant face à l'au moins un dispositif de pompage (30), et
b. la cloison de séparation (41) est réalisée dans la zone de fond en tant que cloison de séparation annulaire (41B), qui entoure une première ouverture de remplissage (60A) du premier réservoir de liquide (40) et qui est entourée par une deuxième ouverture de remplissage (60B) du deuxième réservoir de liquide (40).

9. Distributeur de liquide (10) selon la revendication 8, avec les caractéristiques supplémentaires suivantes :
a. les réservoirs de liquide (40) sont disposés côte à côte au moins dans une zone supérieure des réservoirs de liquide (40) de telle sorte que les deux réservoirs de liquide jouxtent chacun une paroi extérieure commune (43) ainsi qu'une cloison de séparation (41A) non annulaire partant de la paroi extérieure (43), et
b. une structure de cloison de transfert, qui transfère la cloison de séparation non annulaire (41A) dans la cloison de séparation annulaire (41B), est prévue entre la zone de fond et la zone supérieure.

10. Distributeur de liquide (10) selon l'une des revendications précédentes, avec les caractéristiques supplémentaires suivantes :
a. au moins une section partielle inférieure (44) du tuyau d'aspiration (42) est reliée de manière solidaire à un composant de fond de réservoir (16),
de préférence avec au moins une des caractéristiques supplémentaires suivantes :
b. la section partielle inférieure (44) du tuyau d'aspiration (42) est reliée d'un seul tenant au composant de fond de réservoir (16).

11. Distributeur de liquide (10) selon la revendication 10, avec la caractéristique supplémentaire suivante :
a. des surfaces de guidage (47) destinées à placer de manière guidée la section partielle inférieure (44) sur l'ouverture d'aspiration (38), sont prévues sur la section partielle inférieure (44) du tuyau d'aspiration,
de préférence avec au moins une des caractéristiques supplémentaires suivantes :
b. les surfaces de guidage (47) sont prévues sur une multitude de nervures individuelles (46) sous la forme de biseaux d'introduction, et/ou
c. les surfaces de guidage forment une structure de trémie.

12. Distributeur de liquide (10) selon l'une des revendications précédentes, avec au moins une des caractéristiques supplémentaires suivantes :
a. le distributeur de liquide (10) comporte un bouton-poussoir d'actionnement (12), qui peut être déplacé par rapport aux réservoirs de liquide (40), l'au moins un dispositif de pompage (30) étant réalisé pour transporter du liquide hors des réservoirs de liquide (40) vers l'ouverture de décharge (22) par le déplacement du bouton-poussoir d'actionnement (12) par rapport aux réservoirs de liquide (40), et/ou
b. le distributeur de liquide (10) comporte deux dispositifs de pompage (30), qui sont reliés l'un à l'autre mécaniquement de préférence pour l'actionnement simultané ou pour l'actionnement au moins par phase se chevauchant dans le temps, en particulier deux composants de piston (34) des dispositifs de pompage (30) étant couplés l'un à l'autre, et/ou
c. le distributeur de liquide (10) comporte un corps de réservoir principal (14), qui forme d'un seul tenant au moins par endroits une paroi extérieure (43) des deux réservoirs de liquide (40) et forme au moins par endroits la cloison de séparation (41) séparant les réservoirs de liquide (40), et/ou
d. les ouvertures de remplissage (60) sont, dans un état rempli, fermées au moyen d'au moins un composant de bouchon (70), de préférence au moyen d'un composant de bouchon (70) commun pour fermer les deux ouvertures de remplissage (60), et/ou
e. le distributeur de liquide (10) comprend deux dispositifs de pompage (30), qui sont associés chacun à un des deux réservoirs de liquide (40), l'ouverture d'aspiration (38), sur laquelle le tuyau d'aspiration (42) respectif est installé étant prévue de préférence sur une tubulure d'aspiration des dispositifs de pompage (30), et/ou
f. au moins un tuyau d'aspiration (42) est maintenu en supplément au moyen d'un moyen de maintien tenu à distance de l'ouverture d'aspiration (38), en particulier dans la zone d'une cloison enveloppante du réservoir de liquide respectif.

13. Procédé de fabrication et de remplissage d'un distributeur de liquide (10) avec deux réservoirs de liquide (40), avec les caractéristiques suivantes :
a. le distributeur de liquide (10) comporte deux ouvertures de remplissage (60), qui sont prévues chacune dans une zone de fond des réservoirs de liquide (40), et
b. le remplissage des réservoirs de liquide (40) est effectué avec une orientation du distributeur de liquide (10), dans laquelle les ouvertures de remplissage (60) pointent vers le haut, et
c. le remplissage est effectué au moyen au moins d'une tête de remplissage (200), qui dispose d'au moins deux tubulures de remplissage (210), qui rentrent simultanément ou l'une après l'autre dans les ouvertures de remplissage (60) et y distribuent du liquide et remplissent ainsi les réservoirs de liquide (40),
**caractérisé par** la caractéristique supplémentaire suivante :
d. le remplissage est effectué après qu'au moins un composant de fond de réservoir (16), dans lequel l'au moins une ouverture de remplissage (60) est prévue, a été installé au préalable sur un corps de réservoir principal (14), au moins un tuyau d'aspiration (42) du réservoir de liquide (40) étant retiré par pression de l'au moins une ouverture de remplissage (60) au moyen du composant de fond de réservoir (16) dans le cadre de l'installation.

14. Procédé selon la revendication 13, avec au moins une des caractéristiques supplémentaires suivantes :
a. le deuxième réservoir de liquide (40B) entoure le premier réservoir de liquide (40A) au moins dans une zone de fond des réservoirs de liquide (40A, 40B), une cloison de séparation (41) étant réalisée à cet effet au moins par endroits en tant qu'une cloison de séparation annulaire (41B), qui entoure une première ouverture de remplissage (60A) du premier réservoir de liquide (40A) et qui est entourée par une deuxième ouverture de remplissage (60B) du deuxième réservoir de liquide (40B), et
b. le remplissage est effectué au moyen d'une tête de remplissage, qui dispose d'une tubulure de remplissage intérieure (210A) et d'une tubulure de remplissage extérieure (210B), la tubulure de remplissage intérieure (210A) étant disposée à l'intérieur de la tubulure de remplissage extérieure (210B),
de préférence avec la caractéristique supplémentaire suivante :
c. la tubulure de remplissage intérieure (210A) est séparée d'une zone de guidage de liquide de la tubulure de remplissage extérieure (210B) par une cloison intermédiaire à deux coques (212), une coque extérieure (212B) de la cloison intermédiaire (212) étant disposée à l'extérieur de la cloison de séparation (41B) annulaire au cours du remplissage et une coque intérieure (212A) de la cloison intermédiaire (212) étant disposée à l'intérieur de la cloison de séparation (41) annulaire au cours du remplissage.

15. Procédé selon l'une des revendications 13 ou 14, avec au moins une des caractéristiques supplémentaires suivantes :
a. le liquide est distribué par une ouverture de sortie (214) prévue latéralement sur la tubulure de remplissage (210) au cours du remplissage, et/ou
b. la tubulure de remplissage (210) dispose d'une géométrie de refoulement (216) sur sa face frontale distale, sur laquelle le tuyau d'aspiration (42) glisse lorsqu'il rentre dans l'ouverture de remplissage (60).
